# EUROPEAN PATENT APPLICATION

(11) **EP 4 442 818 A1**
(43) Date of publication of application: **09.10.2024**
(21) Application number: 22899855.5
(22) Date of filing: 22.06.2022
(51) Int. Cl.: C12N 9/12

(54) **TAQ POLYMERASE MUTANT, AND PREPARATION METHOD THEREFOR AND USE THEREOF**

(30) Priority: 30.11.2021 CN 202111442628
(71) Applicant: Daan Gene Co., Ltd., Guangzhou, Guangdong 510665 (CN)
(72) Inventor: JIANG, Xiwen, Guangzhou, Guangdong 510665 (CN); LIU, Aishan, Guangzhou, Guangdong 510665 (CN); ZHANG, Wei, Guangzhou, Guangdong 510665 (CN); XIE, Xiaocheng, Guangzhou, Guangdong 510665 (CN); LU, Xuelan, Guangzhou, Guangdong 510665 (CN)
(74) Representative: Viering, Jentschura & Partner mbB Patent- und Rechtsanwälte
(86) International application number: PCT/CN2022/100275
(87) International publication number: WO 2023/098035

(57) **Abstract**

Provided are a Taq polymerase mutant, a preparation method therefor and the use thereof. The Taq polymerase mutant includes an amino acid sequence having at least 70% identity to the amino acid sequence shown in SEQ ID NO:2, wherein the amino acid sequence has mutations occuring at one or more positions selected from the group consisting of: P40, L125, G200, D335, G499, E634, F769 or a combination thereof. The provided Taq polymerase mutant has high amplification activity, can obtain more amplification products compared with wild-type Taq polymerase under the same number of PCR cycles, and moreover, is resistant to whole blood and high salt and has low 5'-3' exonuclease activity.

## Description

### Cross-reference to related application

This patent application claims priority to the Chinese patent application filed on November 30, 2021, with application number 202111442628.3 and title of **"TAQ POLYMERASE MUTANT, AND PREPARATION METHOD THEREOF AND USE THEREOF",** which is hereby incorporated by reference in its entirety.

### Technical Field

The present invention relates to the field of PCR detection, in particular to Taq polymerase mutant and preparation methods and use thereof.

### Background

Taq polymerase is a heat-resistant DNA polymerase derived from the heat-resistant bacterium *Thermus aquaticus*, with a molecular weight of 94 KDa. In the presence of magnesium ions, its optimal reaction temperature is 75-80°C, and its active half-life is 40 minutes at 95°C. It has 5'-3' nucleic acid exonuclease activity. Because of its high temperature resistance, Taq polymerase is widely used in polymerase chain reaction (PCR) and is the enzyme of first choice for nucleic acid amplification and detection. Modern molecular biology testing techniques require increasing sensitivity, precision, and durability of the PCR reaction, and wild-type Taq polymerase cannot fully meet the needs of practical applications.

However, the poor tolerance of wild-type Taq polymerase to extreme conditions (e.g., high salt or whole blood) reduces its range of applicability, accuracy, and sensitivity, and does not allow it to fully meet the requirements of modern molecular bioassay techniques for PCR reactions. Furthermore, when PCR amplification is performed by dye method, the wild-type Taq polymerase in the system has 5'-3' exonuclease activity, which reduces the amplification efficiency in PCR amplification by dye method.

Therefore, it is important to develop a Taq polymerase mutant with low 5'-3' exonuclease activity, which is resistant to whole blood and high salt.

### Summary of invention

It is an object of the present invention to provide a Taq polymerase mutant.

It is another object of the present invention to provide a nucleic acid molecule encoding said Taq polymerase mutant.

It is another object of the present invention to provide a vector.

It is another object of the present invention to provide a host cell.

It is another object of the present invention to provide a method of preparing a Taq polymerase mutant.

It is another object of the present invention to provide a kit containing a Taq polymerase mutant.

In order to solve the above technical problem, a first aspect of the present invention provides a Taq polymerase mutant, wherein the Taq polymerase mutant comprises: an amino acid sequence having at least 70% identity to the amino acid sequence shown in SEQ ID NO:2, wherein the amino acid sequence being mutated at one or more positions selected from the group consisting of: P40, L125, G200, D335, G499, E634, F769, or a combination thereof.

In some preferred embodiments, the amino acid sequence is mutated at any four or more positions selected from the group consisting of P40W, L125I, G200M, D335V, G499K, E634G and F769I.

In some preferred embodiments, the amino acid sequence is mutated at four positions in the following group: D335V, G499K, E634G and F769I; and the amino acid sequence is also mutated at any one or more positions selected from the group consisting of: P40W, L125I and G200M.

In some preferred embodiments, the amino acid sequence is mutated at seven positions in the following group: P40W, L125I, G200M, D335V, G499K, E634G and F769I.

In some preferred embodiments, the Taq polymerase mutant has a resistance to sodium chloride of not less than 70 mM, more preferably not less than 80 mM, more preferably not less than 90 mM, more preferably not less than 100 mM, more preferably not less than 130 mM, more preferably not less than 150 mM, and most preferably not less than 180 mM.

In some preferred embodiments, the Taq polymerase mutant has a resistance to potassium chloride of not less than 100 mM, more preferably not less than 110 mM, more preferably not less than 120 mM, more preferably not less than 130 mM, more preferably not less than 140 mM, more preferably not less than 150 mM, more preferably not less than 180 mM, more preferably not less than 190 mM, and most preferably not less than 200 mM.

In some preferred embodiments, the Taq polymerase mutant has a resistance to EDTA whole blood of not less than 5%, more preferably not less than 7%, more preferably not less than 10%, more preferably not less than 20%, more preferably not less than 30%, more preferably not less than 40%, and most preferably not less than 50%.

In some preferred embodiments, the Taq polymerase mutant has a resistance to heparin whole blood of not less than 1%, more preferably not less than 3%, more preferably not less than 5%, more preferably not less than 10%, more preferably not less than 20%, preferably not less than 30%, and most preferably not less than 35%. SEQ ID NO: 1 the DNA sequence of wild-type Taq polymerase is as follows):

ATGCGTGGCATGCTGCCTCTGTTTGAACCGAAAGGCCGCGTGTTACTGGT
TGATGGCCATCATCTGGCCTATCGTACCTTTCACGCACTGAAGGGTCTGA
CCACAAGCCGTGGCGAGCCGGTTCAGGCCGTGTACGGCTTCGCCAAAAGC
CTGCTGAAAGCCCTGAAAGAAGACGGCGATGCCGTGATCGTGGTTTTCGA
CGCCAAGGCACCGAGCTTTCGTCATGAGGCTTATGGTGGCTATAAAGCCG
GTCGCGCACCGACACCGGAGGATTTCCCGCGCCAGCTGGCCTTAATTAAA
GAGCTGGTGGATCTGTTAGGCCTGGCCCGCCTGGAGGTTCCGGGTTATGA
AGCCGACGACGTGCTGGCCAGCTTAGCCAAGAAGGCAGAGAAAGAGGGC
TATGAGGTGCGCATTCTGACCGCAGACAAGGACCTGTATCAGCTGCTGAG
CGATCGCATTCATGTGCTGCATCCGGAGGGCTACCTGATTACCCCGGCCT
GGCTGTGGGAAAAATATGGTCTGCGCCCGGATCAATGGGCAGATTATCGC
GCACTGACAGGCGATGAAAGCGATAACCTGCCGGGTGTTAAAGGCATTG
GCGAGAAGACAGCACGTAAGCTGCTGGAAGAATgGGGTAgCCTGGAAGC
CCTGTTAAAGAACCTGGACCGTCTGAAGCCGGCAATCCGCGAGAAGATTC
TGGCCCACATGGATGATCTGAAGCTGAGTTGGGATCTGGCCAAAGTTCGC
ACAGACTTACCGCTGGAAGTGGACTTTGCCAAGCGTCGTGAACCGGACCG
CGAACGTCTGCGTGCCTTCTTAGAACGCCTGGAATTCGGTAGCCTGCTGC
ACGAATTTGGTCTGCTGGAAAGCCCGAAAGCCTTAGAAGAAGCTCCGTGG
CCGCCTCCGGAAGGTGCCTTTGTTGGCTTTGTTCTGAGCCGCAAAGAGCC
GATGTGGGCAGATTTACTGGCCCTGGCCGCAGCACGCGGTGGTCGTGTTC
ATCGTGCCCCGGAACCGTATAAAGCCCTGCGCGACCTGAAAGAGGCACG

TGGTCTGTTAGCAAAGGACCTGAGCGTGTTAGCACTGCGTGAAGGTCTGG
GCTTACCGCCTGGTGATGATCCGATGCTGCTGGCCTACCTGCTGGACCCG
AGCAACACCACCCCTGAGGGTGTGGCACGCCGTTATGGTGGTGAATGGAC
CGAGGAAGCAGGTGAACGCGCCGCACTGAGTGAGCGTCTGTTTGCAAAC
CTGTGGGGCCGTCTGGAAGGCGAGGAACGTCTGCTGTGGCTGTACCGCGA
AGTGGAACGCCCGCTGAGCGCCGTTTTAGCCCACATGGAGGCCACCGGCG
TTCGCTTAGATGTGGCCTATCTGCGCGCCCTGAGCCTGGAAGTTGCAGAA
GAAATCGCCCGTCTGGAGGCAGAGGTGTTTCGTTTAGCCGGCCATCCGTT
CAACCTGAATAGCCGCGACCAGCTGGAACGCGTGCTGTTTGATGAACTGG
GCTTACCGGCCATTGGCAAGACCGAGAAGACCGGTAAACGTAGTACCAG
TGCAGCCGTTCTGGAAGCACTGCGTGAAGCCCACCCTATTGTGGAAAAGA
TTCTGCAATATCGCGAGTTAACCAAACTGAAGAGCACCTACATCGATCCT
CTGCCTGATCTGATTCACCCGCGCACCGGTCGTCTGCACACCCGCTTTAAT
CAAACAGCCACAGCCACCGGTCGTCTGAGCAGCAGCGATCCGAACTTAC
AGAACATTCCGGTGCGCACCCCTCTGGGCCAACGCATTCGTCGCGCCTTC
ATTGCCGAAGAAGGCTGGTTACTGGTGGCCCTGGACTACAGCCAGATTGA
GCTGCGTGTTTTAGCCCATCTGAGCGGCGACGAAAACCTGATTCGCGTGT
TTCAAGAGGGCCGCGATATTCATACAGAGACCGCCAGCTGGATGTTTGGT
GTTCCTCGCGAAGCCGTTGATCCGCTGATGCGCCGCGCAGCCAAAACAAT
TAACTTCGGTGTGCTGTATGGTATGAGCGCCCACCGTTTAAGCCAGGAGC
TGGCCATTCCGTATGAAGAGGCCCAGGCATTTATCGAGCGCTACTTCCAG
AGTTTCCCTAAGGTGCGTGCATGGATCGAGAAAACACTGGAGGAAGGTC
GCCGTCGTGGCTACGTGGAAACCCTGTTTGGCCGCCGTCGCTATGTGCCG
GATCTGGAAGCCCGCGTGAAAAGCGTTCGTGAGGCAGCAGAGCGCATGG
CATTTAATATGCCGGTGCAGGGTACCGCAGCCGACCTGATGAAACTGGCA
ATGGTGAAGCTGTTTCCGCGTCTGGAAGAGATGGGCGCCCGCATGTTACT
GCAGGTGCACGACGAACTGGTTCTGGAGGCACCGAAAGAACGTGCCGAA
GCAGTGGCACGCCTGGCCAAAGAGGTTATGGAAGGCGTTTATCCGTTAGC
CGTTCCGCTGGAGGTGGAGGTTGGCATTGGCGAAGACTGGCTGAGTGCAA
AAGAATAA

SEQ ID NO:2 (the amino acid sequence of wild-type Taq polymerase is as follows):

In some preferred embodiments, the amino acid sequence has one or more mutations selected from the group consisting of: P40W, L125I, G200M, D335V, G499K, E634G and F769I.

In some preferred embodiments, the Taq polymerase mutant comprises an amino acid sequence having at least 80% identity with the amino acid sequence shown in SEQ ID NO:1; more preferably, the Taq polymerase mutant comprises an amino acid sequence having at least 90% identity with the amino acid sequence shown in SEQ ID NO:1; more preferably, the Taq polymerase mutant comprises an amino acid sequence having at least 95% identity with the amino acid sequence shown in SEQ ID NO:1.

A second aspect of the present invention further provides a nucleotide molecule, wherein the nucleotide molecule encodes a Taq polymerase mutant described in the first aspect of the present invention.

A third aspect of the present invention further provides a vector comprising the nucleotide molecule described in the second aspect of the present invention.

A fourth aspect of the present invention further provides a host cell, wherein the host cell contains a nucleotide molecule described in the second aspect of the present invention, or its chromosome is integrated with a nucleotide molecule described in the second aspect of the present invention.

In some preferred embodiments, wherein the host cell is a prokaryotic cell, or an eukaryotic cell.

In some preferred embodiments, wherein the prokaryotic cell is E. coli.

In some preferred embodiments, wherein the eukaryotic cell is a yeast cell.

A fifth aspect of the present invention further provides a kit containing a Taq polymerase mutant described in the first aspect of the present invention.

A sixth aspect of the present invention further provides a method of preparing the Taq polymerase mutant described in the first aspect of the present invention, wherein the method comprising the steps of.
(i) culturing the host cell described in the fourth aspect of the present invention under suitable conditions so as to express the Taq polymerase mutant; and
(ii) isolating the Taq polymerase mutant.

A seventh aspect of the present invention also provides uses for the kit described in the fifth aspect of the present invention, which is used for DNA sequencing, DNA labeling, primer extension, amplification, and the like.

The present invention has at least the following advantages over the prior art:
(1) The Taq polymerase mutants of provided by the present invention has high amplification activity, and can obtain more amplification products than the wild-type Taq polymerase at the same number of PCR cycles;
(2) The Taq polymerase mutant provided by the present invention is resistant to both whole blood and high salt;
(3) The Taq polymerase mutant provided by the present invention has low 5'-3' exonuclease activity and is suitable for dye-based PCR systems.

It should be understood that each technical feature of above description and that of the following description (e.g., Examples) may be combined with each other to constitute a new or preferred technical solution. Because of the limitation of space, they will not be repeated herein.

### Description of drawings

One or more embodiments are exemplarily illustrated by the figures in the accompanying drawings, which do not constitute a limitation of the embodiments.
FIG. 1 is a graph of the results of SDS-PAGE identification of the target proteins according to the examples of the present invention.
FIG. 2 is an electropherogram of Taq-aCb protein N1 column purification according to the examples of the present invention.
FIG. 3 is a graph of Taq-aCb protein Q-column purification electrophoresis according to the examples of the present invention.
FIG. 4 is a graph showing the results of wild-type Taq polymerase and Taq-aCb anti-NaCl experiments according to the examples of the present invention.
FIG. 5 is a graph showing the results of Taq-aCb anti-NaCl experiments according to the examples of the present invention.
FIG. 6 is a graph showing the experimental results of wild-type Taq polymerase and Taq-aCb anti-KCl according to the exmples of the present invention.
FIG. 7 is a graph showing the results of the wild-type Taq polymerase and Taq-aCb anti-EDTA blood test according to the examples of the present invention.
FIG. 8 is a graph showing the results of wild-type Taq polymerase and Taq-aCb anti-heparin blood tests according to the examples of the present invention.
FIG. 9 is a graph of RFU-cycling curves according to Example 8 of the present invention.

### Embodiments

The wild-type Taq polymerase has difficulty in amplification under some extreme conditions where its polymerization activity is low, such as high salt, whole blood environments. Although attempts have been made in the prior art to mutate wild-type Taq polymerase to enhance its resistance to extreme conditions, but with little success. Through extensive and in-depth research, the present inventors have screened out Taq polymerase mutants with good performance by applying enzyme site mutation technology. The screened Taq polymerase mutants have good polymerization activity, good resistance to high salt and whole blood, and are suitable for clinical use. In addition, the Taq polymerase mutants of the present invention have low 5'-3' exonuclease activity, which is very suitable for dye-based PCR system.

Some preferred embodiments of the present invention provide a Taq polymerase mutant, which comprises: an amino acid sequence having at least 70% identity to the amino acid sequence shown in SEQ ID NO:2, wherein the amino acid sequence is mutated at one or more positions selected from the group consisting of: P40, L125, G200, D335, G499, E634, F769, or a combination thereof.

In some preferred embodiments, the amino acid sequence has any one or more mutations selected from the group consisting of P40W, L125I, G200M, D335V, G499K, E634G and F769I.

In some preferred embodiments, the amino acid sequence is mutated at four positions in the following group: D335V, G499K, E634G and F769I; and the amino acid sequence is also mutated at any one or more positions selected from the following group: P40W, L125I and G200M.

In some preferred embodiments, the amino acid sequence is mutated at seven positions in the following group: P40W, L125I, G200M, D335V, G499K, E634G and F769I.

In some preferred embodiments, the Taq polymerase mutant has a resistance to sodium chloride of not less than 70 mM, more preferably not less than 80 mM, more preferably not less than 90 mM, more preferably not less than 100 mM, more preferably not less than 130 mM, more preferably not less than 150 mM, and most preferably not less than 180 mM.

In some preferred embodiments, the Taq polymerase mutant has a resistance to potassium chloride of not less than 100 mM, more preferably not less than 110 mM, more preferably not less than 120 mM, more preferably not less than 130 mM, more preferably not less than 140 mM, more preferably not less than 150 mM, more preferably not less than 180 mM, more preferably not less than 190 mM, and most preferably not less than 200 mM.

In some preferred embodiments, the Taq polymerase mutant has a resistance to EDTA whole blood of not less than 5% (the percentage here refers to the percentage of plasma volume to the total volume of the PCR solution system), more preferably not less than 7%, more preferably not less than 10%, more preferably not less than 20%, more preferably not less than 30%, more preferably not less than 40%, and most preferably not less than 50%.

In some preferred embodiments, the Taq polymerase mutant has a resistance to heparin whole blood of not less than 1% (the percentage here refers to the percentage of plasma volume to the total volume of the PCR solution system), more preferably not less than 3%, more preferably not less than 5%, more preferably not less than 10%, more preferably not less than 20%, preferably not less than 30%, and most preferably not less than 35%. SEQ ID NO: 1 (the DNA sequence of wild-type Taq polymerase is as follows):

ATGCGTGGCATGCTGCCTCTGTTTGAACCGAAAGGCCGCGTGTTACTGGT
TGATGGCCATCATCTGGCCTATCGTACCTTTCACGCACTGAAGGGTCTGA
CCACAAGCCGTGGCGAGCCGGTTCAGGCCGTGTACGGCTTCGCCAAAAGC
CTGCTGAAAGCCCTGAAAGAAGACGGCGATGCCGTGATCGTGGTTTTCGA
CGCCAAGGCACCGAGCTTTCGTCATGAGGCTTATGGTGGCTATAAAGCCG
GTCGCGCACCGACACCGGAGGATTTCCCGCGCCAGCTGGCCTTAATTAAA
GAGCTGGTGGATCTGTTAGGCCTGGCCCGCCTGGAGGTTCCGGGTTATGA
AGCCGACGACGTGCTGGCCAGCTTAGCCAAGAAGGCAGAGAAAGAGGGC
TATGAGGTGCGCATTCTGACCGCAGACAAGGACCTGTATCAGCTGCTGAG
CGATCGCATTCATGTGCTGCATCCGGAGGGCTACCTGATTACCCCGGCCT
GGCTGTGGGAAAAATATGGTCTGCGCCCGGATCAATGGGCAGATTATCGC
GCACTGACAGGCGATGAAAGCGATAACCTGCCGGGTGTTAAAGGCATTG
GCGAGAAGACAGCACGTAAGCTGCTGGAAGAATgGGGTAgCCTGGAAGC
CCTGTTAAAGAACCTGGACCGTCTGAAGCCGGCAATCCGCGAGAAGATTC
TGGCCCACATGGATGATCTGAAGCTGAGTTGGGATCTGGCCAAAGTTCGC
ACAGACTTACCGCTGGAAGTGGACTTTGCCAAGCGTCGTGAACCGGACCG
CGAACGTCTGCGTGCCTTCTTAGAACGCCTGGAATTCGGTAGCCTGCTGC
ACGAATTTGGTCTGCTGGAAAGCCCGAAAGCCTTAGAAGAAGCTCCGTGG
CCGCCTCCGGAAGGTGCCTTTGTTGGCTTTGTTCTGAGCCGCAAAGAGCC
GATGTGGGCAGATTTACTGGCCCTGGCCGCAGCACGCGGTGGTCGTGTTC
ATCGTGCCCCGGAACCGTATAAAGCCCTGCGCGACCTGAAAGAGGCACG
TGGTCTGTTAGCAAAGGACCTGAGCGTGTTAGCACTGCGTGAAGGTCTGG
GCTTACCGCCTGGTGATGATCCGATGCTGCTGGCCTACCTGCTGGACCCG
AGCAACACCACCCCTGAGGGTGTGGCACGCCGTTATGGTGGTGAATGGAC
CGAGGAAGCAGGTGAACGCGCCGCACTGAGTGAGCGTCTGTTTGCAAAC
CTGTGGGGCCGTCTGGAAGGCGAGGAACGTCTGCTGTGGCTGTACCGCGA
AGTGGAACGCCCGCTGAGCGCCGTTTTAGCCCACATGGAGGCCACCGGCG
TTCGCTTAGATGTGGCCTATCTGCGCGCCCTGAGCCTGGAAGTTGCAGAA
GAAATCGCCCGTCTGGAGGCAGAGGTGTTTCGTTTAGCCGGCCATCCGTT
CAACCTGAATAGCCGCGACCAGCTGGAACGCGTGCTGTTTGATGAACTGG
GCTTACCGGCCATTGGCAAGACCGAGAAGACCGGTAAACGTAGTACCAG
TGCAGCCGTTCTGGAAGCACTGCGTGAAGCCCACCCTATTGTGGAAAAGA
TTCTGCAATATCGCGAGTTAACCAAACTGAAGAGCACCTACATCGATCCT
CTGCCTGATCTGATTCACCCGCGCACCGGTCGTCTGCACACCCGCTTTAAT
CAAACAGCCACAGCCACCGGTCGTCTGAGCAGCAGCGATCCGAACTTAC
AGAACATTCCGGTGCGCACCCCTCTGGGCCAACGCATTCGTCGCGCCTTC
ATTGCCGAAGAAGGCTGGTTACTGGTGGCCCTGGACTACAGCCAGATTGA
GCTGCGTGTTTTAGCCCATCTGAGCGGCGACGAAAACCTGATTCGCGTGT
TTCAAGAGGGCCGCGATATTCATACAGAGACCGCCAGCTGGATGTTTGGT
GTTCCTCGCGAAGCCGTTGATCCGCTGATGCGCCGCGCAGCCAAAACAAT
TAACTTCGGTGTGCTGTATGGTATGAGCGCCCACCGTTTAAGCCAGGAGC
TGGCCATTCCGTATGAAGAGGCCCAGGCATTTATCGAGCGCTACTTCCAG
AGTTTCCCTAAGGTGCGTGCATGGATCGAGAAAACACTGGAGGAAGGTC

GCCGTCGTGGCTACGTGGAAACCCTGTTTGGCCGCCGTCGCTATGTGCCG
GATCTGGAAGCCCGCGTGAAAAGCGTTCGTGAGGCAGCAGAGCGCATGG
CATTTAATATGCCGGTGCAGGGTACCGCAGCCGACCTGATGAAACTGGCA
ATGGTGAAGCTGTTTCCGCGTCTGGAAGAGATGGGCGCCCGCATGTTACT
GCAGGTGCACGACGAACTGGTTCTGGAGGCACCGAAAGAACGTGCCGAA
GCAGTGGCACGCCTGGCCAAAGAGGTTATGGAAGGCGTTTATCCGTTAGC
CGTTCCGCTGGAGGTGGAGGTTGGCATTGGCGAAGACTGGCTGAGTGCAA
AAGAATAA

SEQ ID NO:2 (the amino acid sequence of wild-type Taq polymerase is as follows):

In some preferred embodiments, the Taq polymerase mutant comprises an amino acid sequence having at least 80% identity with the amino acid sequence shown in SEQ ID NO:1; more preferably, the Taq polymerase mutant comprises an amino acid sequence having at least 90% identity with the amino acid sequence shown in SEQ ID NO:1; more preferably, the Taq polymerease mutant comprises an amino acid sequence having at least 95% identity with the amino acid sequence shown in SEQ ID NO:1.

Some preferred embodiments of the present invention provide a nucleotide molecule, wherein the nucleotide molecule encodes a Taq polymerase mutant described in the first aspect of the present invention.

Some preferred embodiments of the present invention provide a vector comprising the nucleotide molecule described in the second aspect of the present invention.

Some preferred embodiments of the present invention provide a host cell, wherein the host cell contains a nucleotide molecule described in the second aspect of the present invention, or its chromosome is integrated with a nucleotide molecule described in the second aspect of the present invention.

In some preferred embodiments, the host cell is a prokaryotic cell, or an eukaryotic cell.

In some preferred embodiments, the prokaryotic cell is E. coli.

In some preferred embodiments, the eukaryotic cell is a yeast cell.

Some preferred embodiments of the present invention provide a kit containing a Taq polymerase mutant described in the first aspect of the present invention.

Some preferred embodiments of the present invention provide a method of preparing the Taq polymerase mutant described in the first aspect of the present invention, wherein the method comprises the steps of:
(i) culturing the host cell described in the fourth aspect of the present invention under suitable conditions so as to express the Taq polymerase mutant; and
(ii) isolating the Taq polymerase mutant.

Some preferred embodiments of the present invention provide uses for the kit described in the fifth aspect of the present invention, which is used for DNA sequencing, DNA labeling, primer extension, amplification, and the like.

### Terms

As used herein, the term "amino acid" in its broadest sense refers to any compound and/or substance that can be incorporated into a polypeptide chain. In some embodiments, the amino acid has the general structure of H2N-C(H)(R)-COOH. In some embodiments, the amino acid is a naturally occurring amino acid. In some embodiments, the amino acid is a synthetic amino acid; in some embodiments, the amino acid is a D-amino acid; in some embodiments, the amino acid is an L-amino acid. A "standard amino acid" typically refers to any of the twenty standard L-amino acids found in naturally occurring peptides. A "non-standard amino acid" is any amino acid other than a standard amino acid, whether prepared synthetically or obtained from natural sources.

As used herein, "synthetic amino acid" includes chemically modified amino acids, including, but not limited to, salts, amino acid derivatives (e.g., amides) and/or substituents. Amino acids, including carboxyl and/or amino-terminal amino acids in peptides, can be modified by methylation, amidation, acetylation, and/or substitution with other chemicals without adversely affecting their activity. The amino acids may have disulfide bonds. The term "amino acid" is used interchangeably with "amino acid residue" and may refer to free amino acids and/or amino acid residues of peptides. Whether the term refers to free amino acids or peptide residues, it will be apparent from the context in which the term is used. It should be noted that all amino acid residue sequences are represented herein by the left-right orientation of the formula in the conventional direction of amino terminus to carboxy terminus.

As used herein, the term "mutation" refers to the introduction of a change in the parental sequence, including, but not limited to, substitutions, insertions, deletions (including truncations). Consequences of a mutation include, but are not limited to, the occurrence of a new characteristic, property, function, phenotype or trait not found in the protein encoded by the parental sequence. The term "mutant" refers to a modified protein, wherein the modified protein exhibits altered characteristics when compared to the parental protein.

As used herein, the term "% homology" is used herein interchangeably with the term "% identity" and refers to the level of nucleic acid or amino acid sequence identity between nucleic acid sequences encoding any of the polypeptides of the invention or amino acid sequences of the polypeptides of the invention when compared using a sequence comparison program.

As used herein, the term "nucleotide" refers to a monomeric unit of DNA or RNA consisting of a sugar portion (pentose sugar), a phosphate ester, and a nitrogen-containing heterocyclic base. The base is attached to the sugar portion via the glycosidic carbon (the 1' carbon of the pentose sugar), and the combination of the base and the sugar is a nucleoside. When a nucleoside comprises a phosphate group bonded to the 3' or 5'-position of the pentose sugar, it is called a nucleotide. Sequences of operably linked nucleotides are commonly referred to herein as "base sequences" or "nucleotide sequence juxtapositions" and are represented herein in such a form that their left-to-right orientation is in the conventional direction from 5'-terminal to 3'-terminal.

As used herein, the term "vector" refers to a nucleoside construct designed for transferring between different host cells. The term "expression vector" refers to a vector that is capable of incorporating and expressing a heterologous DNA fragment in a foreign cell. Many prokaryotic and eukaryotic expression vectors are commercially available. The selection of suitable expression vectors is within the knowledge of those skilled in the art.

As used herein, the term "host cell" refers to a cell that has been invaded by a target gene, wherein the target gene can invade the cell in the form of a vector (e.g., a virus, chromosome, or plasmid) for replication.

As used herein, the term "chromosomal integration" refers to integration by homologous recombination of multiple copies of the target gene into a sufficiently defined sites.

### Preparation of Taq polymerase mutants

The Taq polymerase gene sequences of the present invention can be obtained by conventional methods available to those of ordinary skill in the art, such as total synthesis or PCR synthesis. A preferred synthesis method is asymmetric PCR. Asymmetric PCR is a method in which a large amount of single stranded DNA (ssDNA) is produced after PCR amplification using an unequal pair of primers. This pair of primers are called a non-restriction primer and a restriction primer, and their ratio is generally 50-100:1. During the first 10-15 cycles of the PCR reaction, the amplification product is mainly double-stranded DNA, but when the restriction primer (a low concentration primer) is depleted, the non-restriction primer (a high concentration primer) guided PCR produces a large amount of single-stranded DNA. Primers for PCR can be appropriately selected based on the sequence information of the present invention disclosed herein and can be synthesized by conventional methods. Amplified DNA/RNA fragments can be separated and purified by conventional methods such as gel electrophoresis.

The Taq polymerase mutants of the present invention can be expressed or produced by conventional recombinant DNA techniques comprising the steps of:
(1) transforming or transducing a suitable host cell with a polynucleotide encoding a protein of the invention, or with a recombinant expression vector containing the polynucleotide;
(2) culturing the host cells in a suitable medium;
(3) isolating and purifying the target protein from the medium or cells to obtain a Taq polymerase mutant.

Methods known to those skilled in the art can be used to construct expression vectors containing DNA sequences encoding the Taq polymerase mutants of the present invention and suitable transcriptional/translational control signals, preferably the commercially available vector: pET28. These methods include in vitro recombinant DNA techniques, DNA synthesis techniques, in vivo recombination techniques and the like. The DNA sequence can be effectively attached to the appropriate promoter in the expression vector to direct mRNA synthesis. The expression vector also includes a ribosome binding site for translation initiation and a transcription terminator. In addition, the expression vector preferably comprises one or more selective marker genes to provide phenotypic traits for selection of host cells for transformation.

The recombinant vector comprises in the 5' to 3' direction: a promoter, a target gene and a terminator. If desired, the recombinant vector may also include the following elements: a protein purification tag; a 3' polynucleotide signal; a nontranslated nucleic acid sequence; a transport and targeting nucleic acid sequence; a selection marker (antibiotic resistance gene, fluorescent protein, etc.); an enhancer; or an operon.

Methods for preparing recombinant vectors are well known to those of ordinary skill in the art. Expression vectors may be a bacterial plasmid, phage, yeast plasmid, plant cell virus, mammalian cell virus, or other vectors. In short, any plasmid and vector can be employed as long as it is capable of replicating and stabilizing in the host.

A person of ordinary skill in the art may employ well known methods for constructing a vector containing a promoter of the present invention and/or a target gene sequence of the present invention. These methods include in vitro recombinant DNA techniques, DNA synthesis techniques, in vivo recombination, and the like.

The expression vectors of the present invention, can be used to transform appropriate host cells to cause the host to transcribe a target RNA or express a target protein. The host cell may be a prokaryotic cell, such as *Escherichia coli*, *Corynebacterium glutamicum*, *Brevibacterium flavum*, *Streptomyces* spp. and *Agrobacterium* spp.; or a lower eukaryotic cell, such as a yeast cell; or a higher eukaryotic cell, such as a plant cell. One of ordinary skill in the art knows how to select the appropriate vector and host cell. Transformation of host cells with recombinant DNA can be performed by conventional techniques known to those of skill in the art. When the host is a prokaryote (e.g., E. coli), it can be treated with CaCl₂. Electroporation can also be used. When the host is eukaryotic, the following DNA transfection methods are available: calcium phosphate co-precipitation, conventional mechanical methods (e.g., microinjection, electroporation, liposome packaging, etc.). Transformation of plants can also be performed using Agrobacterium transformation or gene gun transformation methods, such as leaf disk method, young embryo transformation method, bud immersion method, etc. For transformed plant cells, tissues or organs, they can be regenerated into plants by conventional methods to obtain transgenic plants.

In order to make the objects, technical solutions and advantages of the embodiments of the present invention clearer, the present invention is further elaborated below in connection with specific examples. It should be understood that these examples are used only to illustrate the present invention and are not intended to limit the scope of the present invention. The experimental methods for which specific conditions are not indicated in the following examples, and the experimental methods for which detailed conditions are not indicated in the following examples, are generally in accordance with conventional conditions such as those described in Sambrook. J et al.(ed.) Molecular Cloning: A Laboratory Manual, by U.S.A. (Translated by Huang Peitang et al., Beijing: Science Press, 2002), or in accordance with the conditions recommended by the manufacturer. Unless otherwise indicated, percentages and parts are weight percentages and weight parts. The experimental materials and reagents used in the following examples are available from commercially available sources if not otherwise indicated.

Unless otherwise indicated, technical and scientific terms used herein have the same meanings as are commonly understood by one of ordinary skill in the art to which this application belongs, and it is to be noted that the terms used herein are intended only to describe specific embodiments and is not intended to limit the exemplary embodiments of the present application.

### Example 1: Preparation of Taq polymerase mutants Taq-aCb and Taq-aCb Mut

Steps for the preparation of Taq polymerase mutants.

Step 1: Taq-aCb mutant plasmid was prepared by using wild-type Taq polymerase expression vector as template and QuikChange Lightning Multi-site Mutagenesis Kit, in which the wild-type expression vector was preserved by Guangzhou Daan Gene Co, and the vector was pET28a with His6 tag at the N-terminal. The mutant plasmids were sent for sequencing, and the sequencing results indicated that the mutant vectors were successfully constructed.

### Step 2: Transformation of E. coli BL21(DE3) with the recombinant plasmid

Under ice bath conditions, 1 µ L plasmid was taken and added to 30 µ L *Escherichia coli* BL21 (DE3). After placing in an ice bath for 20 minutes, it was heat shocked in the 42 °C water bath for 45 seconds and immediately transferred to ice for 2 minutes. 400 µ L SOC medium without antibiotics was added, and subjected to 37 °C, 220 rmp shaking culture for 50 minutes. 100 µL of the bacterial solution was spread evenly onto LB plates containing 100 µg/mL kanamycin and incubated overnight in an incubator at 37 °C.

### Step 3: Expression of target protein

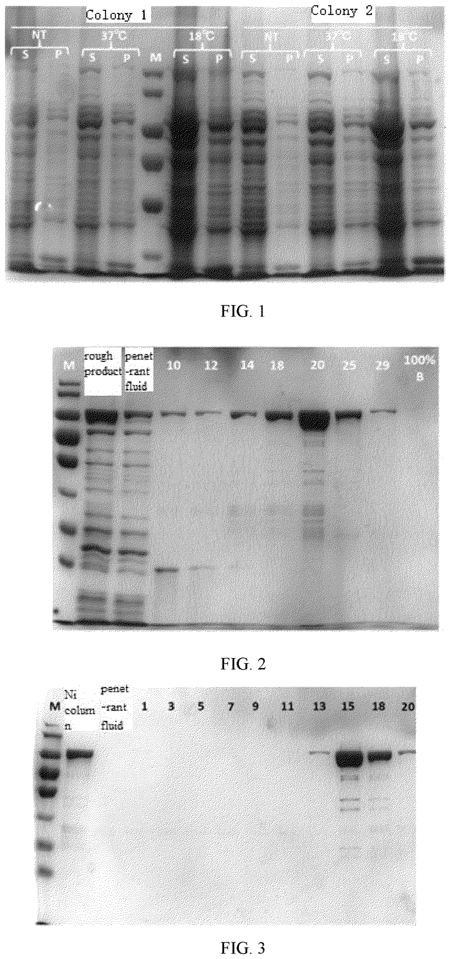

The obtained monoclones in step 2 were picked and aseptically inoculated in TB medium containing 100 µg/mL kanamycin, incubated at 37°C and 220 rpm with shaking until the OD600 was between 0.6 and 0.8, and induced with IPTG (final concentration of 0.1 mM), and left to be incubated overnight at 37°C and 18°C with shaking, respectively. The group without IPTG was used as a control and incubated at 37°C for 3 h. The experiment was repeated once for each group. The samples were ultrasonically broken and analyzed by SDS-PAGE, and the results are shown in Figure 1.

According to Figure 1, a large amount of soluble proteins can be expressed in the supernatant of TB medium at 18 °C, and the amount of soluble proteins accounted for more than 90% of the total amount of target proteins, and the molecular weight of the proteins was about 100Kda.

### Step 4: Purification of Taq-aCb

Shake flasks with 1.5 L of bacteriophage in TB medium were incubated, and the expression conditions were the same as those for the target proteins in step 3). The bacterium were collected by centrifugation, the wet weight of the two proteins was about 30 g. About 4 g of bacteriophage was weighed, 35 ml of Lysis Buffer was added and resuspend on ice. After ultrasonication, centrifugation at 20,000 rpm for 30 min at 4°C was performed, the supernatant was filtered through a 0.22 µm needle filter to obtain the supernatant. The supernatant was subjected to affinity chromatography using a Ni-Column, and the linear elution was carried out using 0-60% Buffer B, and the eluate of the eluted main peak was taken and subjected to ion-exchange elution using a HisTrapTM Q-HP Purification Column, and the linear elution was carried out using 0-60% Buffer C.

The electropherograms are shown in Figures 2 and 3. The expression content of the target protein was calculated. The expression amount of Taq-aCb was 1.46mg/mL. In this case, the concentration of the solution used was as follows:
Buffer B: 50mM Tris, 50mM NaCl, 500mM Imidazole, 5% Glycerol, pH8.5;
Lysis Buffer: 50mM Tris, 300mM NaCl, 5% Glycerol, pH8.5;
Buffer C: 100mM Tris, 1M NaCl, 10% Glycerol, pH8.5.

### Example 2: Test of NaCl resistance of Taq-aCb

Different NaCl concentration gradient solutions were prepared (NaCl concentration gradient configurations are shown in Table 1 below), Buffer A and Buffer B were prepared (formulations of Buffer A and Buffer B are shown in Table 1 below), and the NaCl concentration gradient solutions were formed by mixing Buffer A and Buffer B in different ratios.

**Table 1**

| NaCl concentration /mM | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

The resistance of Taq-aCb to NaCl was determined using wild-type Taq polymerase as a positive control.

The PCR reaction system was prepared according to the formulation in Table 2, in which the RV and M4 primers were purchased from TaKaRa, and the 5X Fast Taq Buffer, pUC19 plasmid were kept by Guangzhou Daan Gene Co. The amount of enzyme added was 5 U per 10 µL of PCR reaction system. pUC19 plasmid was used as the template for amplification experiments, and the PCR products were subjected to 2% agarose gel electrophoresis.

**Table 2**

| reagents | Buffer A | Buffer B |
|---|---|---|
| RV(10pmol) | 1.2µL | 1.2µL |
| M4(10pmol) | 1.2µL | 1.2µL |
| pUC19 plasmid | 6µL | 6µL |
| 5X Fast Taq Buffer | 12µL | 12µL |
| wild type Taq/Taq 2C2/Taq 2C2 Mut | 3 0U | 3 0U |
| 1M NaCl | 6µL | 0µL |
| ddH₂O | 31.6µL | 37.6µL |

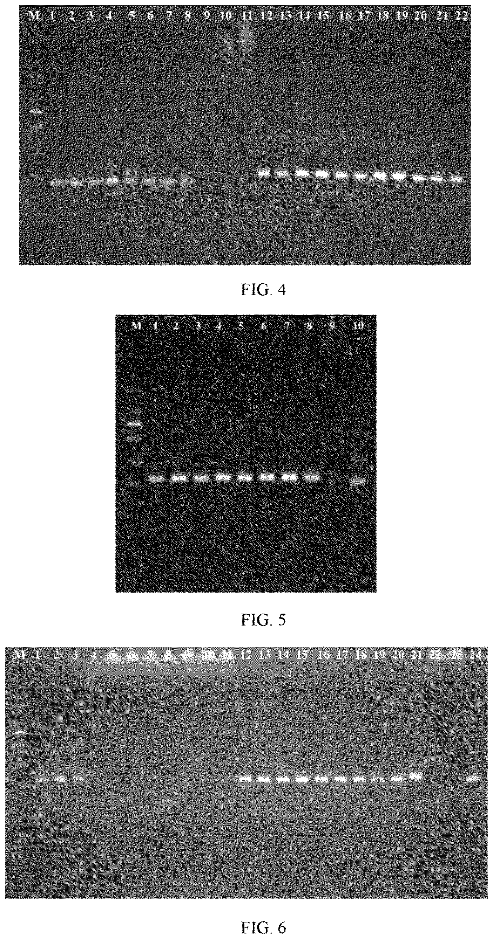

Reaction conditions: 95°C for 2 min, (95°C for 15 s, 44°C for 15 s, 72°C for 1 min) × 30 cycles, 72°C for 1 min, and the electrophoresis results of amplified products are shown in Figure 4.

As shown in Figure 4, 1~11 are the PCR product lanes of wild-type Taq polymerase at 0, 10, 20, 30, 40, 50, 60, 70, 80, 90, and 100 mM NaCl concentration, respectively; 12∼22 are the PCR product lanes of Taq-aCb at 0, 10, 20, 30, 40, 50, 60, 70, 80, 90, and 100 mM NaCl concentration, respectively. It can be seen that Taq-aCb has very good NaCl resistance compared with the wild type.

Continuing to increase the NaCl concentration in the PCR reaction system to 110 mM-190 mM, the electrophoresis results of the amplified products were shown in Figure 5.

As shown in Figure 5, 1∼9 are the PCR product lanes of Taq-aCb at 110, 120, 130, 140, 150, 160, 170, 180, and 190 mM NaCl concentration, respectively, and 10 is the positive control.

According to Fig. 5, the resistance of wild-type Taq enzyme to NaCl was 70 mM, and the resistance of Taq-aCb to NaCl reached 180 mM, so the Taq polymerase mutants had significantly improved NaCl resistance compared with wild-type Taq polymerase.

### Example 3: Test of KCl resistance of Taq-aCb

Different KCl concentration gradient solutions (Table 3) were prepared according to the method in Example 2, wherein NaCl was replaced with KCl in Buffer A.

**Table 3**

| KCl concentration /mM | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

Taq-aCb resistance to KCl was tested according to the same method as in Example 2, and the electrophoresis results of the amplified products are shown in Figure 6.

As in Figure 6, 1~11 are the wild-type Taq polymerase PCR product lanes at KCl concentrations of 50, 80, 100, 150, 160, 170, 180, 190, 200, 250, and 300 mM, respectively; 13∼18 are the PCR product lanes of Taq-aCb at KCl concentrations of 50, 80, 100, 150, 160, 170, 180, 190, 200, 250, and 300 mM, respectively; 12 and 24 are the positive controls.

According to Figure 6, the resistance of wild-type Taq polymerase to KCl was 100 mM, and the Taq mutant Taq-aCb reached resistance to NaCl of 200 mM; thus, the Taq polymerase mutant showed significantly improved resistance to KCl as compared with the wild-type Taq polymerase.

### Example 4: Test of EDTA whole blood resistance of Taq-aCb

Different whole blood concentration gradient solutions (Table 4) were prepared according to the method in Example 2, wherein NaCl in Buffer A was replaced with EDTA whole blood. The volume fraction was calculated according to the volume of blood added to the PCR reaction system.

**Table 4**

| EDTA whole blood concentration/ (V/V) | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

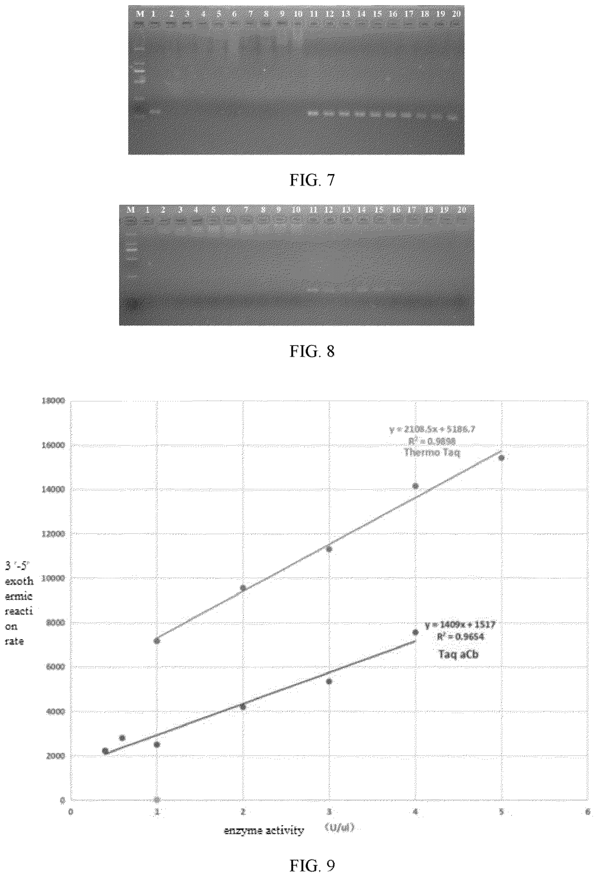

Taq-aCb was tested for resistance to EDTA whole blood according to the same method as in Example 2, and the results of electrophoresis of the amplified products are shown in Figure 7.

As in Figure 7, 1 to 10 are the PCR product lanes of wild-type Taq polymerase at EDTA blood concentration of 5, 10, 15, 20, 25, 30, 35, 40, 45, and 50% (V/V), respectively; 11∼20 are the PCR product lanes of Taq-aCb at EDTA blood concentration of 5, 10, 15, 20, 25, 30, 35, 40, 45, 50% (V/V), respectively.

According to Figure 7, it can be obtained that the resistance of wild-type Taq polymerase to EDTA whole blood was 5% (volume fraction was calculated based on the volume of blood added to PCR as a percentage of the total volume of the PCR system), and Taq mutant enzyme, Taq-aCb, was 45% resistant to EDTA whole blood, so the Taq polymerase mutant has significantly improved its resistance to EDTA whole blood in comparison with wild-type Taq polymerase.

### Example 5: Test of heparin whole blood resistance of Taq-aCb

Different whole blood concentration gradient solutions (Table 5) were prepared according to the method in Example 2, wherein NaCl in Buffer A was replaced with heparin whole blood, and the volume fraction was calculated according to the volume of blood added to the PCR reaction system.

**Table 5**

| Heparin whole blood concentration / (V/V) | 0 | 10 | 20 | 30 | 40 | 50 | 60 | 70 | 80 | 90 | 100 |
|---|---|---|---|---|---|---|---|---|---|---|---|
| Buffer A/µL | 0 | 1 | 2 | 3 | 4 | 5 | 6 | 7 | 8 | 9 | 10 |
| Buffer B/µL | 10 | 9 | 8 | 7 | 6 | 5 | 4 | 3 | 2 | 1 | 0 |

Taq-aCb was tested for resistance to heparin whole blood according to the same method as in Example 2, and electrophoresis results of the amplified products are shown in Figure 8.

As in Figure 8, 1-10 are the PCR product lanes of wild-type Taq polymerase at heparin blood concentration of 5, 10, 15, 20, 25, 30, 35, 40, 45, and 50% (V/V), respectively; 11 to 20 are the PCR product lanes of Taq-aCb at heparin blood concentration of 5, 10, 15, 20, 25, 30, 35, 40, 45, and 50% (V/V), respectively.

According to Figure 8, it can be obtained that the wild-type Taq polymerase has 0% resistance to heparin whole blood (volume fraction was calculated based on the volume of blood added to PCR as a percentage of the total volume of the PCRT system), and the Taq mutant enzyme Taq-aCb has 35% resistance to heparin whole blood, so that the Taq polymerase mutant has a significantly higher resistance to heparin whole blood than the wild-type Taq polymerase.

### Example 6: Taq-aCb 5'-3' exonuclease activity test

5'-3' exonuclease activity was assayed by fluorescent probe PCR with wild-type Taq polymerase (ThermoFisher Scientific) as a positive control, diluted to 1U, 2U, 3U, 4U, 5U, and Taq-aCb was similarly diluted to the same activity concentration. The reaction system was prepared as shown in Table 6 below (wherein 10X Taq Buffer was purchased from TaRaKa):

**Table 6**

| Reagent | mixing volume |
|---|---|
| 10X Taq Buffer | 2.5ul |
| 25mM MgCl2 | 4.5ul |
| 2.5mM dNTPs | 0.2ul |
| Probe2(10pmol) | 0.5ul |
| DNA polymerase test 2(10pmol) | 0.25µl |
| ddH2O | 16.05µL |

PCR reaction conditions: 10 minutes at 95°C, (95°C for 10 seconds, 55°C for 30 seconds, read fluorescence) × 40 cycles. Using the reaction cycle as the X-axis and the fluorescence value (RFU) corresponding to each cycle as the Y-axis, an RFU-cycling graph was made. The known enzyme concentration was used as the X-axis, and the initial slope corresponding to the RFU-cycling graph was used as the Y-axis to fit the standard curve, and the slope of the linear regression equation obtained could indicate the magnitude of 5'-3' exonuclease activity. The results are shown in Figure 9.

According to Figure 9, the 5'-3' exonuclease activity of Taq-aCb was 66% of that of the wild-type Taq polymerase, which was decreased by the mutation compared to the wild type. It was basically consistent with the wild-type Taq polymerase.

It will be understood by one of ordinary skill in the art that the above embodiments are specific embodiments for realizing the present invention, and that various changes can be made thereto in form and detail in practical application without departing from the spirit and scope of the present invention.

## Claims

1. A Taq polymerase mutant, wherein the Taq polymerase mutant comprises: an amino acid sequence having at least 70% identity to the amino acid sequence shown in SEQ ID NO:2, wherein the amino acid sequence is mutated at one or more positions selected from the group consisting of: P40, L125, G200, D335, G499, E634, F769, or a combination thereof.

2. The Taq polymerase mutant according to claim 1, wherein the amino acid sequence has one or more mutations selected from the group consisting of: P40W, L125I, G200M, D335V, G499K, E634G and F769I.

3. The Taq polymerase mutant according to claim 2, wherein the amino acid sequence is mutated at any four or more positions selected from the group consisting of: P40W, L125I, G200M, D335V, G499K, E634G and F769I.

4. The Taq polymerase mutant according to any one of claims 1 to 3, wherein the Taq polymerase mutant has a resistance to sodium chloride of not less than 70 mM;
and/or, wherein the Taq polymerase mutant has a resistance to potassium chloride of not less than 100 mM.

5. The Taq polymerase mutant according to any one of claims 1 to 3, wherein the Taq polymerase mutant has a resistance to EDTA whole blood of not less than 5%;
and/or, wherein the Taq polymerase mutant has a resistance to heparin whole blood of not less than 1%.

6. A nucleotide molecule, wherein the nucleotide molecule encodes the Taq polymerase mutant according to any one of claims 1 to 5.

7. A vector, wherein the vector contains the nucleotide molecule according to claim 6.

8. A host cell, wherein the host cell contains the nucleotide molecule according to claim 7, or its chromosome is integrated with the nucleotide molecule according to claim 5.

9. A kit, wherein the kit contains the Taq polymerase mutant according to any one of claims 1 to 5.

10. A method of preparing the Taq polymerase mutant according to claim 1, wherein the method comprising the steps of:
(i) culturing the host cell according to claim 8 under suitable conditions so as to express the Taq polymerase mutant; and
(ii) isolating the Taq polymerase mutant.
